# EUROPÄISCHE PATENTSCHRIFT

(11) **EP 3 092 139 B1**
(45) Veröffentlichungstag und Bekanntmachung des Hinweises auf die Patenterteilung: **26.06.2019**
(21) Anmeldenummer: 14803050.5
(22) Anmeldetag: 25.11.2014
(51) Int. Cl.: B60H 3/00, A61L 9/03

(54) **VORRICHTUNG ZUM ABGEBEN EINES DUFTSTOFFS UND KRAFTFAHRZEUG MIT EINER DERARTIGEN VORRICHTUNG**
DEVICE FOR DIFFUSING A FRAGRANCE, AND MOTOR VEHICLE COMPRISING A DEVICE OF SAID TYPE
DISPOSITIF DE DIFFUSION D'UN PARFUM ET VÉHICULE AUTOMOBILE POURVU D'UN DISPOSITIF DE CE TYPE

(30) Priorität: 10.01.2014 DE 102014000313
(43) Veröffentlichungstag der Anmeldung: 16.11.2016
(73) Patentinhaber: Audi AG, 85045 Ingolstadt (DE)
(72) Erfinder: BELZ, Karsten, 85051 Ingolstadt (DE); TECHEL, Georg, 01156 Dresden (DE); HOLZER, Michael, 85077 Manching (DE)
(86) Internationale Anmeldenummer: PCT/EP2014/003143
(87) Internationale Veröffentlichungsnummer: WO 2015/104037

(56) Entgegenhaltungen:
- WO-A1-03/019082
- WO-A1-2004/002542
- WO-A1-2008/148154
- WO-A1-2013/111842
- US-A1- 2004 007 787
- US-A1- 2009 035 188

## Beschreibung

Die Erfindung betrifft eine Vorrichtung zum Abgeben eines Duftstoffs, mit einem wenigstens eine Austrittsöffnung aufweisenden Behälter zum Aufnehmen des Duftstoffs und einem dem Behälter zugeordneten elektrischen Heizelement zum Erwärmen des Duftstoffs.

Es sind bereits verschiedene Duftspender vorgeschlagen worden, um einen Duftstoff automatisch abzugeben.

Die DE 103 05 481 A1 schlägt einen Duft- oder Parfümspender vor, der einen Bewegungssensor aufweist. Beim Erfassen einer Person im Umfeld des Duftspenders wird ein Duft gesprüht oder durch ein Heizelement erhitzt und in der Luft verteilt. Optional kann auch ein Gebläse vorgesehen sein. Dieser Duftspender kann einen Stecker für einen Zigarettenanzünderanschluss aufweisen, so dass er in einem Fahrzeug betrieben werden kann.

Aus dem Dokument DE 20 2005 019 989 U1 ist ein Raumbeduftungsgerät mit einem Verdunstungskörper, der ein Heizaggregat aufweist, bekannt. Zur Verteilung des Duftstoffs ist ein Ventilator vorgesehen.

Die DE 10 2005 025 755 A1 offenbart eine Vorrichtung zur Verteilung von Geruchsstoffen in einem Innenraum eines Kraftfahrzeugs. Die Vorrichtung umfasst ein Gehäuse zur Aufnahme des Duftmittels, wobei das Gehäuse mittels wenigstens einer betätigbaren Öffnung selektiv geöffnet oder geschlossen werden kann. Durch zyklisches Betätigen der bewegbaren Öffnung wird eine Beduftung des Fahrzeugs mit kontrollierten Dosen von Duftstoffen ermöglicht.

Aus dem Dokument DE 203 02 097 U ist ein Duftspender für den Innenraum eines Fahrzeugs bekannt, mit einem Stromanschluss, der für die Bordnetzspannung eines Fahrzeugs geeignet ist. Der Duftspender umfasst ein elektrisches Heizelement, das einen mit Duftmaterial gefüllten Behälter langsam oder schnell erhitzt und den Duft frei gibt. Dokument US 2004/0007787A1 zeit eine Vorrichtung zum Abgeben eines Duftstoffs mit einer Temperaturregelung.

Dokumente WO 2004/002542 A1 und WO 2008/148154 zeigen Behälter mit als Folien ausgebildeten Heizelementen.

Bei der Abgabe von Duftstoffen in dem relativ kleinen Innenraum eines Kraftfahrzeugs ist eine exakte Dosierung von hoher Wichtigkeit. Bei einer zu geringen Dosierung wird der Duftstoff nicht oder nicht in ausreichendem Maße wahrgenommen. Eine zu hohe Dosierung wird andererseits von Fahrzeuginsassen als unangenehm oder störend empfunden.

Der Erfindung liegt daher die Aufgabe zugrunde, eine Vorrichtung zum Abgeben eines Duftstoffs anzugeben, die eine genaue Dosierung ermöglicht.

Zur Lösung dieser Aufgabe ist bei einer Vorrichtung gemäß Anspruch 1 erfindungsgemäß vorgesehen, dass dem Behälter ein Temperatursensor zum Erfassen der Temperatur des Duftstoffs zugeordnet ist und dass die Vorrichtung einen Regler zum Regeln des elektrischen Heizelements in Abhängigkeit der erfassten Temperatur aufweist.

Die Erfindung beruht auf der Erkenntnis, dass das Vorsehen eines Heizelements allein nicht ausreicht, um eine genaue Dosierung des Duftstoffs zu bewirken. Der Grund dafür ist, dass die Wahrnehmbarkeit von Duftstoffen stark temperaturabhängig ist. Bei kalten Außentemperaturen, beispielsweise unterhalb des Gefrierpunkts, sind viele Duftstoffe beziehungsweise deren Bestandteile kaum olfaktorisch wahrnehmbar. Andererseits ist die Mehrzahl der Düfte bei ca. 20° C gut riechbar. Bei höheren Temperaturen verdunsten die Bestandteile des Duftstoffs schneller. Duftstoffe bestehen aus einer Vielzahl von unterschiedlichen, leicht flüchtigen bis schwer flüchtigen Anteilen, so dass ein reproduzierbarer Dufteindruck nur bei vergleichbaren Temperaturen erzielt werden kann. Daher wird das elektrische Heizelement der erfindungsgemäßen Vorrichtung mittels des Reglers auf eine festgelegte, konstante Temperatur erwärmt.

Die erfindungsgemäße Vorrichtung kann besonders einfach umgesetzt werden, wenn das Heizelement als Folie ausgebildet ist, die auf wenigstens einer Innenfläche des Behälters angeordnet ist. Gemäß einer Weiterbildung der Erfindung kann die Folie selbst als Behälter ausgebildet sein. Die Folie umfasst einen oder mehrere elektrisch leitfähige Leiterbahnen, die beim Anlegen einer Spannung erwärmt werden.

Um den Energieverbrauch zu verringern, ist es bei der erfindungsgemäßen Vorrichtung vorgesehen, dass der Behälter eine ihn wenigstens teilweise umgebende Wärmedämmschicht aufweist. Erfindungsgemäß ist die Wärmedämmschicht aus einem geschäumten Kunststoff hergestellt.

Eine Weiterbildung der Erfindung sieht vor, dass der Regler dazu ausgebildet ist, den Duftstoff auf eine Temperatur zwischen 10 °C und 30 °C, insbesondere auf näherungsweise 20 °C, zu erwärmen. Durch die Erwärmung auf einen im Wesentlichen konstanten Temperaturbereich, insbesondere auf 20 °C, kann ein reproduzierbarer Dufteindruck erzeugt werden.

Es ist denkbar, dass die erfindungsgemäße Vorrichtung, insbesondere deren Behälter, eine Luftzutrittsöffnung aufweist. Diese Luftzutrittsöffnung ermöglicht es einströmender Luft, die Oberfläche des flüssigen Duftstoffs zu überstreichen und die Vorrichtung über die Austrittsöffnung zu verlassen.

Daneben betrifft die Erfindung ein Kraftfahrzeug mit einer Vorrichtung der beschriebenen Art.

Bei dem erfindungsgemäßen Kraftfahrzeug wird es bevorzugt, dass das elektrische Heizelement der Vorrichtung an das Bordnetz des Kraftfahrzeugs angeschlossen ist.

Es ist besonders günstig, wenn bei dem erfindungsgemäßen Kraftfahrzeug die Vorrichtung derart mit einer Lüftungs- und/oder Klimaanlage des Kraftfahrzeugs gekoppelt ist, dass der in dem Behälter angeordnete Duftstoff durch einen Luftstrom beaufschlagbar ist. Der Luftstrom durchströmt dabei den Behälter der Vorrichtung, wodurch der Duftstoff verdunstet und sich im Innenraum des Kraftfahrzeugs verteilt.

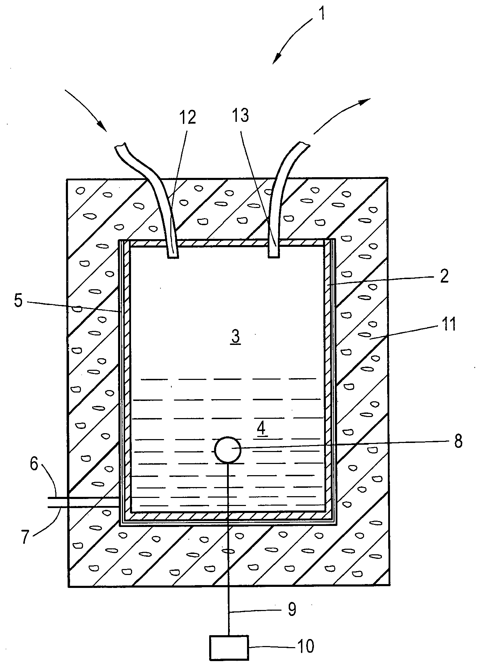

Die Erfindung wird nachfolgend anhand eines Ausführungsbeispiels unter Bezugnahme auf die Zeichnung erläutert. Die Zeichnung ist eine schematische Darstellung und zeigt eine erfindungsgemäße Vorrichtung zum Abgeben eines Duftstoffs in einer geschnittenen Ansicht, die in einem Kraftahrzeug eingesetzt werden kann.

Die in der Zeichnung gezeigte Vorrichtung 1 umfasst einen Behälter 2, der in dem dargestellten Ausführungsbeispiel als Hohlzylinder ausgebildet ist. Ein Hohlraum 3 im Inneren des Behälters 2 ist mit einem flüssigen Duftstoff 4 gefüllt. Zur Temperierung des Duftstoffs 4 weist der Behälter 2 ein als Folie ausgebildetes Heizelement 5 auf. Es sind auch andere Ausführungen möglich, bei denen das Heizelement sich an der Innenseite des Behälters befindet. Das Heizelement 5 umfasst schematisch dargestellte elektrische Anschlussleitungen 6, 7, die mit dem Bordnetz eines Kraftfahrzeugs verbunden sind. Beim Anlegen einer Spannung an die Anschlussleitungen 6, 7 wird das Heizelement 5 erwärmt. Im Inneren des Behälters 2 ist ein Temperatursensor 8 angeordnet, der sich an einer beliebigen Stelle befinden kann. Bevorzugt wird dabei die Anbringung des Temperatursensors 8 im Zentrum des Behälters 2 oder am Boden oder an der Seitenwand. Der Temperatursensor 8 erfasst die Temperatur des Duftstoffs 4, über eine elektrische Leitung 9 ist der Temperatursensor 8 mit einem Regler 10 verbunden. Der Regler 10 dient zur Regelung des elektrischen Heizelements 5. Die Regelung kann beispielsweise durch Regeln des das Heizelement 5 durchfließenden Stroms erfolgen, alternativ kann das Heizelement auch mit einem konstanten Strom beaufschlagt werden, wobei die Stromzufuhr durch den Regler 10 intermittierend erfolgt. In dem dargestellten Ausführungsbeispiel ist der Regler 10 dazu ausgebildet, den Duftstoff 4 auf eine Temperatur von 20 °C zu erwärmen.

In der Zeichnung erkennt man, dass der Behälter 2 von einer Wärmedämmschicht 11 umgeben ist, die zur thermischen Isolierung des Duftstoffs 4 dient und aus einem geschäumten Kunststoff hergestellt ist.

An der Oberseite des Behälters 2 ist eine Luftzutrittsöffnung 12 vorgesehen, daneben ist an der Oberseite des Behälters 2 eine Austrittsöffnung 13 vorhanden. Die beiden Öffnungen sind voneinander beabstandet, sie können auch an gegenüberliegenden Seiten des Behälters 2 angeordnet sein.

Die Luftzutrittsöffnung 12 umfasst eine Rohrleitung, über die die Vorrichtung 1 mit einer Lüftungs- und Klimaanlage des Kraftfahrzeugs verbunden ist. Die Lüftungs- und Klimaanlage erzeugt einen Luftstrom, der über die Rohrleitung und die Luftzutrittsöffnung 12 den Behälter 2 zugeführt wird, so dass die Oberfläche des flüssigen Duftstoffs 4 dem Luftstrom ausgesetzt ist. Beim Verdunsten des erwärmten Duftstoffs 4 wird dieser von der Luftströmung aufgenommen und verlässt die Vorrichtung 1 über die Austrittsöffnung 13.

Durch die Temperaturregelung des Duftstoffs 4, bei der die festgelegte Temperatur die Regelgröße ist, entsteht immer der gleiche Dufteindruck, unabhängig von der Außentemperatur oder der Innentemperatur des Kraftfahrzeugs.

## Patentansprüche

1. Vorrichtung (1) zum Abgeben eines Duftstoffs (4), mit einem wenigstens eine Austrittsöffnung (13) aufweisenden Behälter (2) zum Aufnehmen des Duftstoffs (4) und einem dem Behälter (2) zugeordneten elektrischen Heizelement (5) zum Erwärmen des Duftstoffs (4), wobei dem Behälter (2) ein Temperatursensor (8) zum Erfassen der Temperatur des Duftstoffs (4) zugeordnet ist und die Vorrichtung (1) einen Regler (10) zum Regeln des elektrischen Heizelements (5) in Abhängigkeit der erfassten Temperatur aufweist,
**dadurch gekennzeichnet,**
**dass** das Heizelement (5) als Folie mit einer oder mehreren elektrisch leitfähigen Leiterbahnen, die durch Anlegen einer Spannung erwärmbar sind, ausgebildet ist, wobei die Folie auf einer Außenfläche des Behälters (2) oder auf wenigstens einer Innenfläche des Behälters (2) angeordnet ist, wobei der Behälter (2) eine ihn wenigstens teilweise umgebende Wärmedämmschicht (11) aufweist, wobei die Wärmedämmschicht (11) aus einem geschäumten Kunststoff hergestellt ist.

2. Vorrichtung nach Anspruch 1,
**dadurch gekennzeichnet,**
**dass** der Regler (10) dazu ausgebildet ist, den Duftstoff (4) auf eine Temperatur zwischen 10 °C und 30 °C, insbesondere auf näherungsweise 20 °C, zu erwärmen.

3. Vorrichtung nach Anspruch 1 oder 2,
**dadurch gekennzeichnet,**
**dass** der Behälter (2) eine Luftzutrittsöffnung (12) aufweist.

4. Kraftfahrzeug mit einer Vorrichtung (1) nach einem der Ansprüche 1 bis 3.

5. Kraftfahrzeug nach Anspruch 4,
**dadurch gekennzeichnet,**
**dass** das elektrische Heizelement (5) der Vorrichtung (1) an das Bordnetz des Kraftfahrzeug angeschlossen ist.

6. Kraftfahrzeug nach Anspruch 4 oder 5,
**dadurch gekennzeichnet,**
**dass** die Vorrichtung (1) derart mit einer Lüftungs- und/oder Klimaanlage des Kraftfahrzeugs gekoppelt ist, dass der in dem Behälter (2) angeordnete Duftstoff (4) mit einen Luftstrom beaufschlagbar ist.

## Claims

1. Device (1) for diffusing a fragrance (4), having a receptacle (2) comprising at least one discharge opening (13) for holding the fragrance (4), and an electrical heating element (5) associated with the receptacle (2) for heating the fragrance (4), wherein the receptacle (2) is assigned a temperature sensor (8) for detecting the temperature of the fragrance (4) and the device (1) comprises a controller (10) for controlling the electrical heating element (5) in accordance with the detected temperature,
**characterised in that**
the heating element (5) is configured as a film comprising one or a plurality of electrically conductive tracks which can be heated by applying a voltage, wherein the film is arranged on an outer surface of the receptacle (2) or on at least one inner surface of the receptacle (2), wherein the receptacle (2) has a heat insulating layer (11) at least partially surrounding it, wherein the heat insulating layer (11) is made from a foamed plastic.

2. Device according to claim 1,
**characterised in that**
the controller (10) is configured such that the fragrance (4) is to be heated to a temperature between 10°C and 30°C, in particular to approximately 20°C.

3. Device according to claim 1 or 2,
**characterised in that**
the receptacle (2) comprises an air inlet opening (12).

4. Motor vehicle with a device (1) according to any one of claims 1 to 3.

5. Motor vehicle according to claim 4,
**characterised in that**
the electrical heating element (5) of the device (1) is connected to the on-board power supply.

6. Motor vehicle according to claim 4 or 5,
**characterised in that**
the device (1) is coupled to a ventilation and/or air conditioning system such that the fragrance (4) arranged in the receptacle (2) can be acted on by an airflow.

## Revendications

1. Dispositif (1) pour la diffusion d'un parfum (4) avec un récipient (2) présentant au moins une ouverture de sortie (13) pour recevoir le parfum (4) et un élément électrique chauffant (5) affecté au récipient (2) pour chauffer le parfum (4), dans lequel un capteur de température (8) est affecté au récipient (2) pour recueillir la température du parfum (4) et le dispositif (1) présente un régulateur (10) pour régler l'élément électrique chauffant (5) en fonction de la température recueillie,
**caractérisé en ce que** :
l'élément chauffant (5) se présente sous la forme d'un film avec une ou plusieurs pistes électroconductrices qui peuvent être chauffées par application d'une tension, dans lequel le film est agencé sur une surface externe du récipient (2) ou sur au moins une surface interne du récipient (2), dans lequel le récipient (2) présente une couche calorifuge (11) l'entourant au moins en partie, dans lequel la couche calorifuge (11) est constituée d'une matière plastique expansée.

2. Dispositif selon la revendication 1,
**caractérisé en ce que** :
le régulateur (10) est conçu de manière à chauffer le parfum (4) à une température comprise entre 10 °C et 30 °C, en particulier aux environ de 20 °C.

3. Dispositif selon la revendication 1 ou 2,
**caractérisé en ce que** :
le récipient (2) présente une ouverture d'entrée d'air (12).

4. Véhicule automobile avec un dispositif (1) selon l'une quelconque des revendications 1 à 3.

5. Véhicule automobile selon la revendication 4,
**caractérisé en ce que** :
l'élément électrique chauffant (5) du dispositif (1) est connecté au réseau de bord du véhicule automobile.

6. Véhicule automobile selon la revendication 4 ou 5,
**caractérisé en ce que** :
le dispositif (1) est couplé à une installation d'aération et/ou de climatisation du véhicule automobile en sorte que le parfum (4) disposé dans le récipient (2) soit alimenté par un courant d'air.
